## Europäisches Patentamt

## European Patent Office

## Office européen des brevets

(19)

(11) Veröffentlichungsnummer: **0 208 213 B1**

# (12) EUROPÄISCHE PATENTSCHRIFT

(45) Veröffentlichungstag der Patentschrift:
18.10.89

(51) Int. Cl.⁴: **A 61 K 9/32**

(21) Anmeldenummer: **86108776.5**

(22) Anmeldetag: **27.06.86**

(54) Arzneimittelumhüllung.

(30) Priorität: **08.07.85 DE 3524337**

(43) Veröffentlichungstag der Anmeldung:
**14.01.87 Patentblatt 87/3**

(45) Bekanntmachung des Hinweises auf die Patenterteilung:
**18.10.89 Patentblatt 89/42**

(84) Benannte Vertragsstaaten:
**AT BE CH DE FR GB IT LI NL SE**

(56) Entgegenhaltungen:
EP-A- 0 152 038
DE-A- 1 467 843

DIE PHARMAZIE, Band 39, Nr. 4, 1984, Seiten 233-237, VEB Verlag Volk und Gesundheit, Berlin, DE; J. TRAUE et al.: "Über den Einsatz von Polyacrylatdispersionen zur Sprühverkapselung von Phenobarbital"

(73) Patentinhaber: **Röhm GmbH, Kirschenallee Postfach 4242, D-6100 Darmstadt 1 (DE)**

(72) Erfinder: **Lehmann, Klaus, Dr., Schillerstrasse 85, D-6101 Rossdorf 1 (DE)**
Erfinder: **Petereit, Hans-Ulrich, Lortzingstrasse 22, D-6100 Darmstadt (DE)**

## Beschreibung

Die Erfindung bezieht sich auf Umhüllungsmittel für Arzneimittel in Form einer wäßrigen Dispersion, auf die damit hergestellten Arzneimittel und daraus hergestellte Arzneimittelkapseln.

### Stand der Technik

Überzugsmittel für Arzneitabletten, enthaltend einen wasserunlöslichen, filmbildenden Kunststoff in Form einer wäßrigen Dispersion und eine wasser- oder alkalilösliche Substanz, sind aus der DE-C 1 617 351 bekannt. Für magensaftresistente Überzüge wird eine alkalilösliche Substanz mitverwendet, die im alkalischen Milieu aus dem Tablettenüberzug herausgelöst wird und Poren hinterläßt, durch die danach der Wirkstoff ausdiffundieren kann. Als alkalilösliche Substanz werden z.B. Fettsäuren vorgeschlagen. Die Wirkstofffreisetzung beginnt, wenn die Tablette in ein wäßriges Milieu von einem PH-Wert eintritt, bei dem sich die alkalilösliche Substanz auflöst.

Aus der gleichen Druckschrift ist es auch bekannt, in den Überzug makromolekulare, wasserlösliche Substanzen einzubringen, wie Polyäthylenglykole, die unabhängig vom pH-Wert des Milieus löslich sind. Der Einsatz von alkalilöslichen makromolekularen Substanzen war noch nicht bekannt, jedoch war zu erwarten, daß sie ebenso wie niedermolekulare alkalilösliche Substanzen die Diffusionsdurchlässigkeit des Überzugs bei einem pH-Wert herbeiführen, bei dem sie löslich sind.

Die Kombination eines dispergierten Acrylesterpolymerisats mit nicht wasserlöslichen Celluloseäthern ist aus der EP-A 52 075 bekannt. Sie ergibt Arzneimittelüberzüge, die eine verzögerte Wirkstofffreisetzung bewirken.

Gemäß DE-A 3 127 237 wird eine wäßrige Dispersion eines magensaftresistenten Überzugsmittels mit Polyäthylenglykol und Polyvinylpyrrolidon kombiniert und ergibt Arzneimittelüberzüge, die im Darmsaft schnell zerfallen.

Thiele und Pflegel haben in «Pharmazie» 1981, S. 858 - 859 und 1983, S. 43 - 45 über Tablettenüberzüge aus wäßrigen Dispersionen, enthaltend 98 bis 99 Gew.-% eines schwach carboxylgruppenhaltigen hydrophoben Acryl-/Methacrylesterpolymerisats und 1 bis 2 Gew.-%, jeweils auf den Gesamtpolymerisatgehalt bezogen, eines hydrophilen Mischpolymerisats aus Acryl-/Methacrylsäure und Acryl-/Methacrylsäurealkylester, berichtet. Der Zusatz des letztgenannten Polymermaterials bewirkt zwar eine Steigerung der Diffusionsdurchlässigkeit, aber keine schnelle Wirkstofffreigabe im alkalischen Bereich. Bei Zusätzen von mehr als 2 Gew.-% des hydrophilen Polymerisats erwies sich der Überzug als instabil. Eine Abhängigkeit der Durchlässigkeit vom pH-Wert wurde nicht festgestellt. Vielmehr erfolgt die Wirkstofffreigabe allein durch Diffusion durch die porenfreie Membran, weitgehend unabhängig vom pH-Wert.

Aus der DE-C 2 135 073 sind Arzneimittelüberzugsdispersionen von Polymeren, die 10 bis 55 Gew.-% carboxylgruppenhaltige Monomerbausteine enthalten, bekannt. Sie ergeben Überzüge auf Arzneiformen, die sich bereits im schwach alkalischen Milieu der oberen Darmabschnitte bei hohem Carboxylgruppengehalt schnell, bei niedrigem Carboxylgruppengehalt langsamer auflösen.

Die Polymeren sind verhältnismäßig hart und spröde und wenig dehnbar. Dies kann sich z.B. beim Verpressen von magensaftresistent oder retardierend überzogenen Granulaten störend bemerkbar machen, da die Umhüllung platzen und der Wirkstoff bereits vorzeitig im Magen freigesetzt werden kann.

Aus der DE-C 2 157 435 sind Arzneimittelkapseln aus diesen Polymeren bekannt. Durch Zusatz von Weichmachungsmitteln, wie Citronensäureestern, läßt sich die Sprödigkeit soweit vermindern, daß die Herstellung, Füllung und Handhabung der Kapseln möglich ist, jedoch war eine weitere Erhöhung der Dehnbarkeit wünschenswert.

Auch nach EP-B 56 825 ist der Zusatz von Weichmachern bei der Herstellung von Kapseln aus wäßrigen Dispersionen der erwähnten Art vorgesehen.

### Aufgabe und Lösung

Die Elastizität und Dehnbarkeit von magensaftresistenten und retardierenden Arzneimittelumhüllungen, die aus wäßrigen Dispersionen herstellbar sind, sollte auch ohne zwingenden Einsatz von üblichen Weichmachungsmitteln verbessert werden.

Es wurde gefunden, daß sich die Reißdehnung, gemessen nach DIN 53 455 von Polymerfilmen, die beim Trocknen von wäßrigen Dispersionen carboxylgruppenhaltiger Emulsionspolymerisate entstehen, durch den Zusatz begrenzter Mengen eines Latex von nicht wasserlöslichen, filmbildenden Polymeren, von hoher Dehnbarkeit wesentlich erhöhen läßt. Ebenso läßt sich eine Steigerung der Zugfestigkeit feststellen, was vor allem im Bereich der erst mäßig erhöhten Reißdehnung von Bedeutung ist.

Nachfolgend werden für Filme, die aus Latexmischungen von

A) einem Mischpolymerisat aus 50 Gew.-% Methacrylsäure und 50 Gew.-% Äthylacrylat und

B) einem Mischpolymerisat aus 33 Gew.-% Methylmethacrylat und 67 Gew.-% Äthylacrylat in verschiedenen Mischungsverhältnissen erzeugt worden sind, die Meßwerte der Reißdehnung (Dehnungslänge beim Bruch, bezogen auf die Länge im ungedehnten Zustand) und der Zugfestigkeit (Zugkraft beim Dehnungsbruch) angegeben:

| Mischungsverhältnis A : B (Trockengewicht der Polymeren) | Reißdehnung % | Zugfestigkeit N/mm$^2$ |
|---|---|---|
| 10 : 0 | 14 | 10 |
| 9 : 1 | 72 | 22 |
| 8 : 2 | 93 | 17 |
| 7 : 3 | 290 | 6,2 |
| 0 : 10 | 600 | 7,5 |

Es ist überraschend, daß bereits geringe Zusätze des filmbildenden, hoch dehnbaren Polymers eine wesentliche Verbesserung der Zugfestigkeit und Reißdehnung bewirken. Die Magensaftresistenz bleibt durch den Zusatz unverändert. Auch läßt sich die schnelle oder retardierte Wirkstofffreisetzung im Darmsaft durch Veränderung des Carboxylgehaltes in dem Polymeren A nach Bedarf ebenso leicht einstellen, wie in Abwesenheit des Polymers B.

*Anwendung*

Die neuen Umhüllungsmitteldispersionen eignen sich für die Herstellung von Arzneiformen mit verzögerter Wirkstoffabgabe insbesondere solchen, die den Magen unverändert passieren und ihren Wirkstoff erst im Darmbereich endgültig freisetzen sollen, unter Verwendung einer wäßrigen Überzugsmitteldispersion. Die Wirkstoffabgabe kann hierbei je nach Dicke der Umhüllung, dem Mischungsverhältnis der Polymeren und sonstigen Formulierungsbestandteilen schon im sauren pH-Bereich des Magens durch Diffusion durch die zwar noch intakte aber mehr oder weniger permeable Polymerschicht beginnen, nimmt dann aber oberhalb pH 5 durch die Auflösungstendenz des Polymeren A zu. Ist schließlich ein großer Anteil des Polymeren A aus dem Filmverband herausgelöst, zerfällt die Umhüllung und gibt den Inhalt an Arzneistoffen frei. Wenn die Wirkstoffabgabe im Bereich des Magens kleiner als 5 % der Gesamtdosis ist, spricht man von magensaftresistenten Arzneiformen, sonst von Zubereitungen mit verzögerter bzw. retardierter Wirkstoffabgabe. Bei der Anwendung von Retard-Arzneiformen in anderen Körperhöhlen (z.B. Rektum, Vagina, Mund, Nase, Gehörgang) bzw. am Auge oder auf der Haut kann die gewünschte Permeabilität oder Zerfallsneigung der Polymerumhüllung auch dem in diesen Bereichen anzutreffenden Milieu, insbesondere dem pH-Wert und den Elektrolytkonzentrationen, entsprechend angepaßt und notfalls noch durch Mitverwendung von Puffersubstanzen in der Arzneiformulierung stabilisiert werden.

*Das carboxylgruppenhaltige Polymere*
liegt in Form von in einer Wasserphase dispergierten Latexteilchen vor. Die Herstellung derartiger Latizes ist in der DE-C 2 135 073 und der DE-A 3 134 222 beschrieben. Es ist jedoch nicht erforderlich, daß die Dispersion dieses Polymeren allein filmbildend ist. Daher können hartmachende Comonomere, wie niedere Methacrylsäureester, einen höheren Anteil des Polymerisats bilden, als wenn die Dispersion allein als Überzugsmittel verwendet würde.

Das carboxylgruppenhaltige Polymere muß wenigstens in einem Teil des pH-Bereichs zwischen 5 und 8 wasserlöslich sein, kann aber im unteren Teil dieses Bereichs noch wasserunlöslich sein. Damit es in Wasser dispergierbar ist, muß es wenigstens unter pH 5 wasserunlöslich sein. Oberhalb pH 8 ist es in der Regel wasserlöslich, jedoch hat diese Eigenschaft keine Bedeutung für die Erfindung. Die wäßrige Dispersion der Latexteilchen A und B hat auf jeden Fall einen pH-Wert, bei dem das carboxylgruppenhaltige Polymere ungelöst ist.

In der Regel wird das Polymere durch radikalische Emulsionspolymerisation von Vinylmonomeren in wäßriger Phase hergestellt. Ein Teil der Vinylmomeren, vorzugsweise 10 bis 70, insbesondere 25 bis 55 Gew.-%, enthält wenigstens eine Carboxylgruppe. Bevorzugte Vinylmonomere dieser Art sind Acryl- und Methacrylsäure, jedoch sind auch Malein-, Fumar-, Croton- oder Itakonsäure einsetzbar. Der verbleibende Teil der Vinylmonomeren ist frei von Carboxylgruppen und kann aus Estern der genannten Carbonsäuren, insbesondere den Alkylestern mit 1 bis 8 C-Atomen im Alkylrest, Acryl- oder Methacrylnitril, Styrol, α-Methylstyrol, Vinyltoluol, Vinylchlorid oder Vinylestern von Fettsäuren, wie Vinylacetat, bestehen. Hydrophilierende neutrale Comonomere, wie Acrylamid, Methacrylamid, Vinylpyrrolidon oder Hydroxyalkylester der Acryl- oder Methacrylsäure können in begrenzten Mengen am Aufbau der Emulsionspolymerisate beteiligt sein. Sie führen zu einer gewissen Diffusionsdurchlässigkeit des Überzugs schon bei niedrigen pH-Werten, was nur in Sonderfällen erwünscht ist.

Der Anteil der carboxylgruppenhaltigen Monomeren wird jeweils so bemessen, daß das Polymerisat im Bereich zwischen pH 5 und 8 wasserlöslich ist und der Wirkstoff beim gewünschten pH-Wert freigesetzt wird. Wird das Polymere A allein untersucht, so erweist sich seine Lösungsgeschwindigkeit vom Carboxylgruppengehalt abhängig. Hydrophile Comonomere wirken sich steigernd, hydrophobe Comonomere verzögernd auf die Lösungsgeschwindigkeit aus. Als wasserlöslich werden im Sinne der Erfindung solche Polymeren angesehen, die sich in Form von 10 - 20 μm dicken Filmen in künstlichem Darmsaft von pH 7,5 unter mäßigem Rühren innerhalb von höchstens 60 min auflösen. Verzögerte Wasserlöslichkeit wirkt sich in den erfindungsgemäßen Überzügen durch eine Verschiebung des Zerfallspunktes zu höheren pH-Werten aus.

Die Lösungsgeschwindigkeit hängt auch vom Molekulargewicht ab. Der Gewichtsmittelwert des Molekulargewichts liegt im allgemeinen nicht über 500 000 und vorzugsweise im Bereich von 50 000 bis 300 000.

*Das filmbildende Polymere*
liegt gleichfalls in Form von wäßrig dispergierten Latexteilchen vor und wird ebenfalls bevorzugt durch radikalische Emulsionspolymerisation geeigneter Vinylmonomerer erzeugt. Filmbildende wäßrige Polymerdispersionen sind in großer Zahl im Handel und ihre Herstellung ist aus zahlreichen Druckschriften allgemein bekannt.

Als filmbildend werden die Emulsionspolymerisate bezeichnet, wenn sie unter den üblichen Anwendungsbedingungen von wäßrigen Arzneimittelüberzugsdispersionen einen zusammenhängenden, an dem überzogenen Kern fest haftenden Film ergeben. In der Regel tritt eine solche Filmbildung schon bei Raumtemperatur ein, jedoch kann auch einen höhere Trocknungstemperatur angewandt werden. Das filmbildende Polymere wird in der Regel so ausgewählt, daß es in Form des wäßrigen Latex eine Mindestfilmbildungstemperatur nach DIN 53 787 nicht über 60 °C, vorzugsweise nicht über 40 °C hat. Wesentlich für die Steigerung der Dehnbarkeit des aus den Polymeren A und B gebildeten Films ist eine hohe Dehnbarkeit des filmbildenden Polymeren B. Sie soll bei Raumtemperatur mindestens 50 % und vorzugsweise mehr als 100% betragen. Diese Bedingung ist im allgemeinen erfüllt, wenn die dynamische Einfriertemperatur ($T_{\lambda max}$-Wert nach DIN 53 445) nicht über 80°C, vorzugsweise nicht über 40°C liegt.

Die Emulsionspolymerisate können aus den gleichen Vinylmonomeren aufgebaut sein wie die carboxylgruppenhaltigen Polymeren, jedoch liegt der Anteil an carboxylgruppenhaltigen Comonomeren wesentlich niedriger oder fehlt völlig. Auch hydrophile Comonomere sind nur in so begrenzter Menge einpolymerisiert, daß das Polymere im physiologischen pH-Bereich des Magen-Darm-Traktes von 1 bis 8 nicht wasserlöslich ist. Wasserlöslichkeit erst oberhalb pH 8 würde die Brauchbarkeit des Polymeren nicht ausschließen.

Die hohe Dehnbarkeit setzt einen hohen Anteil von «weichen» Comonomeren am Aufbau des Polymerisats voraus.

Hierzu gehören in erster Linie die Alkylester der Acrylsäure. Sie bilden in der Regel einen Anteil im Bereich von 40 bis 80 Gew.-% des Polymerisats. Handelsübliche Arzneimittelüberzugsdispersionen für die Herstellung unlöslicher Überzüge auf Diffusionstabletten erfüllen diese Forderungen und sind für die Zwecke der Erfindung besonders geeignet.

*Das Umhüllungsmittel*

wird zweckmäßig durch Mischen der Latizes vom Typ A und B hergestellt, wobei das Mischungsverhältnis der Gewichtsanteile der beiden Polymertypen über 60 : 40 und unter 95 : 5, vorzugsweise zwischen 70 : 30 und 85 : 15 liegt. Es versteht sich von selbst, daß die zu mischenden Dispersionen miteinander verträglich sein müssen. Beispielsweise dürfen sie keine entgegengesetzt geladenen Emulgiermittel enthalten, wenn es dadurch zur Koagulation käme.

Das Mischungsverhältnis beeinflußt die Mindestfilmbildungstemperatur des Überzugsmittels. Sie liegt zwischen den Mindestfilmbildungstemperaturen der Ausgangslatizes der Polymeren A und B. Wenn sie unerwünscht hoch liegt, kann sie durch einen Zusatz von Filmbildungshilfsmitteln, die entweder als Weichmacher im Film verbleiben oder als flüchtiges Lösungsmittel bei der Trocknung entweichen, erniedrigt werden. Beispiele solcher Filmbildungshilfsmittel sind Äthylen- oder Propylenglykol, Glycerin, Citronensäureester und Polyäthylenglykole.

Mit zunehmendem Anteil des Polymeren A im Verhältnis zum Polymeren B steigt oberhalb des Ph-Wertes, bei dem überhaupt eine Wirkstoffdurchlässigkeit auftritt, die Freisetzungsgeschwindigkeit für den in der überzogenen Arzneiform eingeschlossenen Wirkstoff. Während bei niedrigen Gehalten des Polymeren A die Überzugsmembran zwar durchlässig wird, aber erhalten bleibt, zerfällt sie bei höheren Gehalten an Polymeren A bald nach Erreichen des pH-Wertes, bei dem sie durchlässig wird, vor allem wenn der überzogene Kern durch Quellung eine Sprengwirkung entfaltet.

Die Polymeren A und B bilden in der Regel einen Anteil von 10 bis 40 Gew.-% des wäßrigen Überzugsmittels. Der Rest entfällt auf Wasser, darin gelöste Emulgiermittel und eventuelle Zusätze.

Neben den Polymeren A und B kann das Überzugsmittel übliche gelöste oder suspendierte Hilfsmittel und Zusätze enthalten. Außer den schon erwähnten Filmbildungshilfsmitteln kommen als Zusätze z.B. Konservierungsmittel, Verdickungsmittel, Glanzmittel, Farbstoffe und Pigmente in Betracht.

Die Viskosität des flüssigen Überzugsmittels liegt zweckmäßigerweise im Bereich von 10 bis 1000 cP. Sein pH-Wert liegt unter 6, in der Regel zwischen 2 und 5.

*Die überzogene Arzneiform*

Erfindungsgemäß können alle Arzneiformen umhüllt werden, die den eingeschlossenen Wirkstoff bei einem vorbestimmten pH-Wert gezielt freisetzen sollen. Die Wirkstoffabgabe kann zur Verbesserung der Verträglichkeit geringfügig über einen Zeitraum von 10 - 60 min retardiert werden oder für eine Langzeittherapie in Körperhöhlen oder auf der Haut bis zu einigen Monaten dauern.

Es können Tabletten, Drageekerne, Pillen, Granulate, Kristalle, Pulver gegebenenfalls sogar Gelatinekapseln überzogen werden. Man kann auch Granulate oder Pellets unter Verwendung der erfindungsgemäßen Mittel nach einem üblichen Granulierverfahren herstellen. Die Granulate können ihrerseits überzogen oder zu Tabletten verpreßt werden.

Die Umhüllungsverfahren entsprechen denjenigen, die bei herkömmlichen Arzneimittelüberzugsmitteldispersionen angewendet werden können. Bevorzugt sind Kesseldragierverfahren, bei denen das Überzugsmittel portionsweise oder kontinuierlich auf die rotierenden Arzneiformen aufgegossen oder aufgesprüht wird. Dabei wird in der Regel Warmluft zum Trocknen aufgeblasen. Vorteilhaft ist weiterhin das Wirbelschichtverfahren, das vorzugsweise bei einer Lufttemperatur von 40 bis 60°C durchgeführt wird.

Die Arzneistoffe können aber auch in die Polymeren eingebettet werden, indem man z.B. die Wirkstoffe in den Latizes löst oder suspendiert und die Mischungen zu Filmen auftrocknen läßt oder auch arzneistoffhaltige andere Materialien wie Papier, Textilien, Pflaster u.a. mit den Polymerfilmen beschichtet. Hierbei können die Filme auch zunächst getrennt von den Arzneistoffen hergestellt und erst in einem späteren Arbeitsgang zusammengebracht werden.

Die pH-abhängige Freigabecharakteristik tritt bei Schichtdicken von 10 bis 30 μm besonders deutlich in Erscheinung. Bei der Beschichtung von Granulaten, Partikeln und Kristallen entspricht dies einem Lackauftrag von 10 bis 20 Gew.-%, bei Tabletten, Dragees oder Kapseln einem Lackauftrag von 3 bis 5 Gew.-%, jeweils bezogen auf das Gewicht des überzogenen Kerns. Unterhalb dieses Bereiches ist mit einer zunehmend zeitabhängigen statt pH-abhängigen Freigabe durch Diffusion, oberhalb dieses Bereichs mit zunehmend verzögerter Freigabe beim pH-Wert des Auflösungsbereichs zu rechnen.

*Arzneimittelkapseln*

Eine bevorzugte Anwendung der neuen Dispersionen ist die Herstellung von Arzneimittelkapseln.

Zur Herstellung von Steckkapseln werden in an sich bekannter Weise polierte Docken in die Dispersion eingetaucht und gewünschtenfalls solange abtropfen lassen, bis die zurückbleibende Schicht beim Trocknen die gewünschte Schichtdicke ergibt. Dann läßt man die Schicht vorzugsweise unter Rotieren der Docken im Warmluftstrom bei 25 bis 50°C trocknen. Die getrocknete Schicht wird in üblicher Weise am oberen Rand beschnitten, von der Docke abgestreift und jeweils zwei Teile passender Größe zu einer Kapsel zusammengesteckt. Die Kapselwanddicke bzw. die Dicke der nach dem Eintauchen und Abtropfen auf der Docke zurückbleibende Schicht kann durch die Viskosität der Dispersion beeinflußt werden. Die Viskosität läßt sich auf verschiedene Weise erhöhen, nämlich durch Steigerung des pH-Wertes bis in den beginnenden Quellungsbereich des carboxylgruppenhaltigen Polymeren A, durch Zusatz von quellend wirkenden flüchtigen oder nicht-flüchtigen Weichmachungsmitteln, wie Äthylenglykol, Propylenglykol oder Glycerin oder durch Zusatz von wasserlöslichen hochmolekularen Verdickungsmitteln, wie Celluloseäthern, Polyäthylenglykolen, Polyacrylsäure oder Polyvinylpyrrolidon. Durch diese Zusätze wird eine Thixotropierung der Dispersion erreicht. Es können auch Verdickungszusätze der verschiedenen Typen gleichzeitig angewendet werden. Die Kapselwanddicke kann auch durch mehrmaliges Tauchen und Trocknen der Docken gesteigert werden.

Eine andere Art von Kapseln läßt sich nach dem Folienschweißverfahren herstellen. Zu diesem Zweck wird auf eine rotierende erwärmte Walze oder ein endloses Band eine gleichmäßige Schicht der Dispersion aufgetragen und zu einer Folie von etwa 50 bis 150 µm Dicke getrocknet. Wenn man eine Walze mit näpfchenförmigen Einsenkungen verwendet, läßt sich eine mit entsprechenden Einsenkungen versehene Folie unmittelbar herstellen. Andernfalls können solche Einsenkungen nachträglich in der Wärme, beispielsweise bei 60 bis 100°C mittels eines entsprechenden Walzenpaares eingeprägt werden. In diese Einsenkungen werden flüs´.ige oder feste Arzneistoffe in dosierter Menge eingefüllt. Eine zweite, in der Regel auf die gleiche Weise erzeugte Folie wird darübergelegt und die mit Arzneistoff gefüllten Einsenkungen mit einem heißen Werkzeug unter gleichzeitigem Verschweißen der beiden Folien am Rande der Einsenkungen zu einzelnen Kapseln ausgestanzt.

Die Herstellung der Kapseln aus magensaftresistentem Material ist wesentlich rationeller und zuverlässiger als das Umhüllen von Hartgelatinekapseln mit einem magensaftresistenten Überzug.

*Beispiel 1*

*Herstellung zerfallender Tabletten mit gesteuerter Wirkstoffabgabe*

1440 g eines Emulsionspolymerisats aus gleichen Teilen Methacrylsäure und Äthylacrylat mit einem Trockensubstanzgehalt von 30% wurden mit 960 g eines Emulsionspolymerisats aus 1 Gew.-Teil Methylmethacrylat und 2 Gew.-Teilen Äthylacrylat und einem Trockensubstanzgehalt von ebenfalls 30% vermischt. Getrennt davon wurden 360 g Talkum in 2730 g Wasser suspendiert und in dieser Suspension 18 g Tween 80 (Polyoxyethylensorbitanmonostearat) aufgelöst.

Die Latexmischung wurde nun mit der wäßrigen Suspension vermischt und zum Überziehen von 6 kg Acetylsalicylsäurekristallen einer Korngröße zwischen 0,2 und 0,8 mm verwendet. Dazu wurde die Sprühsuspension in ein Vorratsgefäß überführt und unter ständigem Rühren mittels einer Schlauchpumpe einer Luftdrucksprühpistole zugeführt. Die Kristalle wurden nun in einer Wirbelschichtapparatur der Fa. Glatt (WSG 5) bei einer Zulufttemperatur von 50°C aufgewirbelt, wobei die Warmluft von unten einströmte und die Sprühpistole im oberen Bereich des Wirbelbettes mit nach unten gerichtetem Sprühstrahl montiert wurde. Die Latexmischung wurde nun mit einer Sprühgeschwindigkeit von 33 g/min bei gleichbleibender Warmluftzufuhr aufgesprüht, wobei sich eine Ablufttemperatur von ca. 35°C einstellte. Der Lackauftrag war nach 170 min beendet. Es wurde nun noch 10 min unter schwachem Wirbeln Warmluft zur Nachtrocknung eingeblasen und die überzogenen Kerne auf Bleche ausgebreitet und im Umlufttrockenschrank bei 40°C über Nacht getrocknet.

Die überzogenen Kerne wurde nach der USP XXI (Paddle-Methode) geprüft, wobei die Umdrehungszahl des Rührers 100 UpM betrug. Dabei betrug die Wirkstoffabgabe innerhalb von 2 h in künstlichem Magensaft weniger als 5%. Anschließend wurde der Magensaft gegen künstlichen Darmsaft pH 6,8 ausgetauscht. Die Wirkstoffabgabe betrug hierin nach 1 h mehr als 75%, nach 2 h mehr als 85%.

547 g der überzogenen Kristalle wurden mit 300 g mikrokristalliner Cellulose (Avicel PH 101) und 150 g Maisstärke sowie 3 g Magnesiumstearat vermischt und auf einer Exzenterpresse mit einer Preßkraft von ca. 15 kN zu Tabletten von 10 mm Durchmesser und 600 mg Gesamtgewicht gepreßt. Diese Tabletten zerfielen im USP-Paddlegerät bei 100 U/min innerhalb von 5 min, wobei die unbeschädigten überzogenen Partikel aus dem Tablettenverband wieder freigesetzt wurden. Die Wirkstoffabgabe in künstlichem Magensaft war innerhalb von 2 h nach wie vor kleiner als 5%, nach Wechsel in künstlichem Darmsaft waren innerhalb 1 h mehr als 75% und nach 2 h mehr als 85% des Wirkstoffes aufgelöst.

*Beispiel 2*

*Herstellung umhüllter Partikel mit gesteuerter Wirkstoffabgabe*

150 g eines Polymerpulvers, hergestellt durch Sprühtrocknung eines Emulsionspolymerisats aus gleichen Teilen Methacrylsäure und Äthylacrylat wurden in 300 g Wasser suspendiert und unter tropfenweisem Rühren mit 50,5 g 1 N Natronlauge versetzt. Der nach weiterem Rühren von ca. 30 min entstandene Latex wurde mit 250 g eines Emulsionspolymerisats aus 1 Gew.-Teil Methylmethacrylat, 2 Gew.-Teilen Äthylacrylat mit einem Trockengehalt von 30% vermischt. Getrennt davon wurden in 750 g Wasser 3,3 g Polyoxyäthylensorbitanmonostearat (Tween 80) gelöst, darin 100 g Talkum suspendiert und zuletzt noch unter Rühren 15 g Acetylcitronensäuretriäthylester emulgiert. In dieses Gemisch wurde die obige Latexmischung unter Rühren eingegossen und noch 10 min bis zur vollständigen Homogenisierung gerührt.

In einem Laborwirbelschichtgerät (Uniglatt) wurden 1 kg Acetylsalicylsäurekristalle einer Korngrösse zwischen 0,3 und 0,8 mm in einem Luftstrom von 41°C aufgewirbelt und insgesamt 600 g der Sprühsuspension über eine Schlauchpumpe der Sprühdüse zugeführt, die im oberen Teil des Wirbelbettes mit nach unten gerichtetem Sprühstrahl montiert war. Mit einem Düsendurchmesser von 1 mm und einem Sprühdruck von 1,8 bar wurde die Suspension nun mit einer Geschwindigkeit von 3,5 g/min kontinuierlich eingesprüht. Dabei stellte sich eine Ablufttemperatur von ca. 25°C ein, der gesamte Sprühprozeß war nach 4 h beendet. Die Kristalle wurden noch ca. 5 min im Luftstrom schwach gewirbelt und dann über Nacht an der Luft nachgetrocknet.

Die Wirkstoffabgabe im USP-Paddlegerät wurde wie im Beispiel 1 untersucht: in künstlichem Magensaft wurden nach 1 h 2,7% und nach 2 h 4,0% freigesetzt. Nach dem Wechsel betrug die Freigabe in künstlichem Darmsaft nach einer weiteren Stunde 62% und nach 2 h 85%.

### Beispiel 3

#### Herstellung von Leerkapseln

5,6 g Polyacrylsäure (Carbopol 934 PH) wurden in 180 g Wasser suspendiert und dazu die wäßrigen Lösungen von 15 g Polyäthylenglykol 6000 in 60 g Wasser und 5 g Polyoxysorbitanmonostearat (Tween 80) in 10 g Wasser zugegeben und verrührt. 240 g eines Emulsionspolymerisats aus gleichen Gewichtsteilen Methacrylsäure und Äthylacrylat mit 30% Trockengehalt wurden mit 105 g eines Emulsionspolymerisats aus 1 Teil Methylmethacrylat und 2 Teilen Äthylacrylat mit ebenfalls 30% Trockengehalt vermischt und in die zuerst hergestellte Zubereitung eingerührt. Diese Mischung wurde in einen evakuierbaren Rührkessel überführt und unter vermindertem Druck von etwa 250 mbar eine verdünnte Ammoniaklösung eingetropft, die aus 10 g 3 N Ammoniak und 64 g Wasser hergestellt worden war.

Die oben hergestellte Zubereitung wurde in eine Tauchwanne überführt. Mit einem für die Herstellung von Gelatinekapseln gebräuchlichen Tauchmechanismus wurden zylindrische VA-Stahl-Docken, wie sie ebenfalls bei der Herstellung von Hartgelatinekapseln gebräuchlich sind, in die Dispersion getaucht, wobei die Tauchzeit 2 sek betrug und der anhaftende Flüssigkeitsfilm anschließend an der Luft bei Raumtemperatur und ca. 60% rel. Luftfeuchtigkeit getrocknet wurde. Die erhaltenen Kapseln hatten eine Wandstärke von ca. 200 µm und Abmessungen entsprechend der Kapselgröße 1. Zur Prüfung der Magensaftresistenz wurde in das Kapselunterteil 250 mg einer 0,3%igen Milchzucker/Methylenblau-Pulvermischung eingefüllt und die Kapsel durch Andrücken des Oberteils verschlossen. Bei der Prüfung auf Magensaftresistenz wurde innerhalb von 2 h in künstlichem Magensaft (0,1 N Salzsäure) im USP-Zerfallstester geprüft. In dieser Zeit war nur ein leichtes Anquellen der Kapselwand zu erkennen, ohne daß Flüssigkeit in die Kapsel eindrang oder ein Zerfall eintrat. Nach anschließendem Wechsel in künstlichem Darmsaft BP 80, pH 6,8 wurde der Inhalt innerhalb von 15 min freigesetzt, nach 60 min war die gesamte Kapselwand zerfallen.

### Beispiel 4

#### Herstellung von Matrixtabletten durch Direktverpressung

Aus einer Latexmischung 7 : 3 der Emulsionspolymerisate aus 70 Teilen Methacrylsäure und 30 Teilen Acrylsäuremethylester und des neutralen Emulsionspolymerisats, das in Beispiel 1 eingesetzt wurde, wurde durch Sprühtrocknung ein Pulver hergestellt. 170 g dieses Polymerpulvers wurden mit 600 g Theophyllin und 100 g mikrokristalliner Cellulose (Avicel PH 102), 20 g Talk (DAB) und 10 g Magnesiumstearat (DAB) vermischt und unter einem Druck von 18 kN zu Matrixtabletten von 12 mm Durchmesser und 450 mg Gewicht verpresst, entsprechend 300 mg Theophyllin pro Tablette.

Diese Tabletten wurden im USP-Zerfallstester in einem Phosphatpuffer pH 5 etwa 8 h beobachtet und die Wirkstoffabgabe gemessen. Die Tabletten zerfallen in dieser Zeit teilweise durch langsame Erosion von der Oberfläche her und geben den Wirkstoff mit einer Geschwindigkeit von 15 - 20% der Wirkstoffdosis pro Stunde kontinuierlich ab.

### Beispiel 5

400 g einer Latexmischung aus den Emulsionspolymerisaten des Beispiels 4 im Verhältnis 85 : 15 mit 15% Polymertrockengehalt wurden in einem Zwangsmischer in eine Mischung aus 400 g Diprophyllin und 129,8 g Kalziumhydrogenphosphat (Emcompress) eingetragen. Die feuchte Masse wurde durch ein Sieb mit 1,5 mm Maschenweite gedrückt, das entstehende Granulat bei 40°C im Warmlufttrockenschrank über Nacht getrocknet auf eine Restfeuchte unter 2% und noch einmal durch ein Sieb mit 1,5 mm Maschenweite gesiebt. Anschließend wurden daraus nach Zusatz von 6,6 g Talkum und 3,6 g Magnesiumstearat unter produktionsüblichen Bedingungen Tabletten gepreßt. Der Durchmesser der Preßlinge liegt bei 12 mm, die Bruchfestigkeit (Pharmatest PTB Type 301) bei 150 N, das Gesamtgewicht bei 600 mg entsprechend 400 mg Dipropyhllin pro Einzeldosis. Im USP-Paddlegerät zeigten diese Tabletten ein langsames Aufquellen und einen Zerfall innerhalb von 4 h. In dieser Zeit wurde der Wirkstoff mit einer Geschwindigkeit von 25 - 30% der Wirkstoffdosis pro Stunde freigegeben.

### Beispiel 6

67 g einern Latexmischung aus den Emulsionspolymerisaten des Beispiels 1 im Verhältnis 9 : 1 (Trockensubstanzgehalt 30%) werden mit 190 g gereinigten Wassers gemischt, in einem Zwangsmischer mit 300 g feinem Theophyllinpulver (Teilchengröße unter 110 µm) vermengt und entsprechend Beispiel 5 aufgearbeitet.

Dem getrockneten Granulat werden zugemischt: 24 g mikrokristalline Cellulose (Avicel PH 102), 4 g Talkum und 2 g Magnesiumstearat.

Diese Granulatmasse wird zu Tabletten verpreßt mit 350 mg Gewicht, einer Bruchfestigkeit von 110 bis 150 N und 10 mm Durchmesser.

Im USP-Dissolutions-Testgerät nach Methode 2 (Paddlegerät) setzen diese Tabletten nach 2 h in künstlichem Magensaft ca. 30% der eingesetzten Dosis frei, während nach Wechsel in künstlichen Darmsaft von pH 6,8 (BP 1980) nach weiteren 3 h mehr als 90% der gesamten Theophillinmenge in Lösung gegangen ist.

*Beispiel 7*

Mit 176,3 g einer Latexmischung (30% Trockensubstanz) im Verhältnis 60 : 40 aus Emulsionspolymerisaten aus gleichen Teilen Methacrylsäure und Äthylacrylat einerseits, sowie aus 1 Gew.-Teil Methacrylsäuremethylester und 2 Gew.-Teilen Acrylsäureäthylester andererseits werden 35 g einer 20%-igen wäßrigen Lösung von Polyäthylenglykol 6000 und 140 g gereinigtes Wasser gleichmäßig vermischt und darin 3,2 g Tranylcyprominsulfat gelöst.

Dieser Ansatz wird durch ein 0,1-mm-Sieb auf einer planen Fläche von 1085 cm² ausgebreitet und bei Raumtemperatur in einem Umlufttrockenschrank getrocknet. Es entsteht ein etwa 500 µm dicker Film, der 3 mg Wirkstoff pro cm² enthält.

In einem Freigabeversuch mit einer Pufferlösung von pH 5,5 (8,82 g $NaH_2PO_4 \cdot H_2O$ und 0,39 g $Na_2HPO_4$ pro Liter) bei 34°C wurde der Wirkstoff aus diesem Film wie folgt verzögert freigesetzt:

nach 12 Stunden 0,7 mg/cm²
nach 24 Stunden 1,0 mg/cm²
nach 40 Stunden 1,4 mg/cm².

Solche Arzneizubereitungen eignen sich insbesondere für eine Anwendung auf der Haut.

*Beispiel 8*

*Einbettung von Wirkstoffpulver durch Verschweißen*

Es wurden Filme aus der in Beispiel 3 beschriebenen Dispersionsmischung hergestellt. Die Filme hatten eine Dicke von 400 µm und wurden zu Bahnen von 160 mm Breite geschnitten. Diese Filme wurden auf einer Tiefziehmaschine wie folgt verarbeitet: Aus der planen Folie werden durch Druckluft von etwa 6 bar bei einer Temperatur von 80 bis 100°C ellipsoide Vertiefungen geformt, in die ca. 200 mg der in Beispiel 3 beschriebenen Milchzucker-Methylenblau-Mischung gefüllt wird. Eine gleichartige plane Folie wird darüber gelegt und die gefüllten Vertiefungen bei einer Temperatur von etwa 100°C am Rand unter Druck mit dieser verschweißt und ausgestanzt. Die verschweißten Kapseln weisen folgende Freigabecharakteristik auf, wenn sie entsprechend dem in Beispiel 3 beschriebenen Verfahren geprüft werden: kein Eindringen von Flüssigkeit in künstlichem Magensaft über 2 h und Freigabe des Kapselinhalts nach maximal 10 min, Zerfall der Kapselwand nach maximal 40 min.

*Beispiel 9*

*Überziehen von Tabletten*

Eine nach Beispiel 1 hergestellte Latexmischung wurde mit der dort beschriebenen Talkum-Suspension vermischt. 10 kg Acetylsalicylsäure-Tabletten von 12 mm Durchmesser und einem Wirkstoffgehalt von je 500 mg wurden in einem Dragierkessel bei einer Rotationsgeschwindigkeit von 30 U/min mit der obigen Suspension unter Warmluftzufuhr (40 bis 50°C) kontinuierlich besprüht, wobei eine Druckluftspritzpistole mit 1 mm Düsendurchmesser und ein Sprühdruck von 1,2 bar verwendet wurde. Die Sprühzeit betrug insgesamt 3,5 Stunden. Die Tabletten wurden über Nacht an der Luft bei Raumtemperatur getrocknet. Die Überzugsschicht hat eine Dicke von 5 bis 20 µm.

**Patentansprüche**

1. Wäßrige Dispersion eines Umhüllungsmittels für Arzneistoffe, enthaltend dispergierte Latexteilchen
A) eines zwischen pH 5 und 8 wasserlöslichen, carboxylgruppenhaltigen Polymeren, und
B) eines nicht wasserlöslichen, filmbildenden Polymeren von hoher Dehnbarkeit,
dadurch gekennzeichnet, daß das Gewichtsverhältnis der Gesamtmenge der Latexteilchen A und B über 60 : 40 und unter 95 : 5 liegt.

2. Wäßrige Dispersion nach Anspruch 1, dadurch gekennzeichnet, daß die Polymeren A und B Emulsionspolymerisate von Vinylmonomeren sind.

3. Wäßrige Dispersion nach Anspruch 2, dadurch gekennzeichnet, daß das carboxylgruppenhaltige Polymere 10 bis 70 Gew.-% Einheiten der Acryl- und/oder Methacrylsäure enthält.

4. Wäßrige Dispersion nach den Ansprüchen 2 und 3, dadurch gekennzeichnet, daß die Polymeren A und B einpolymerisierte Einheiten von Alkylestern der Acryl- und/oder Methacrylsäure enthalten.

5. Wäßrige Dispersion nach den Ansprüchen 1 bis 4, gekennzeichnet durch eine dynamische Einfriertemperatur des filmbildenden Polymeren zwischen 10 und 60°C.

6. Wäßrige Dispersion nach den Ansprüchen 1 bis 5, gekennzeichnet durch einen Gehalt an den Polymeren A und B von 10 bis 40 Gew.-%.

7. Verwendung einer wäßrigen Dispersion nach den Ansprüchen 1 bis 6, zum Umhüllen von Arzneiformen.

8. Verwendung einer wäßrigen Dispersion nach den Ansprüchen 1 bis 6, zur Herstellung von Arzneimittelkapseln.

9. Verfahren zur Herstellung magensaftresistent oder retardierend überzogener Arzneiformen durch Überziehen mit einer wäßrigen Arzneimittelüberzugsdispersion und Trocknen, dadurch gekennzeichnet, daß eine wäßrige Dispersion gemäß den Ansprüchen 1 bis 6 aufgebracht wird.

10. Arzneiform, enthaltend eine Umhüllung, die die Polymeren A und B gemäß Anspruch 1 im Gewichtsverhältnis über 60 : 40 und unter 95 : 5 enthält.

11. Arzneiform nach Anspruch 10, gekennzeichnet durch einen Überzug einer Dicke von 10 bis 30 Micrometer auf Granulaten, Partikeln oder Kristallen.

12. Arzneiform nach Anspruch 10, gekennzeichnet durch einen Überzug auf Granulaten, Partikeln oder Kristallen mit einem Lackauftrag von 10 bis 20 Gew.-%, bezogen auf das Gewicht der überzogenen Arzneiformen.

13. Arzneiform nach Anspruch 10, gekennzeichnet durch einen Überzug auf Tabletten, Dragees oder Kapseln mit einem Lackauftrag von 3 bis 5 Gew.-%, bezogen auf das Gewicht der überzogenen Arzneiformen.

14. Magensaftresistente Arzneimittelkapseln, bestehend aus einem Gemisch der Polymeren A und B gemäß Anspruch 1 im Gewichtsverhältnis über 60 : 40 und unter 95 : 5.

## Claims

1. Aqueous dispersion of a coating composition for medicaments, comprising dispersed latex particles

A) of a carboxyl group-containing polymer which is water-soluble at between pH 5 and 8, and

B) a water-insoluble film-forming polymer of high extensibility,

characterized in that the weight ratio of the total quantity of latex particles A and B is above 60 : 40 and below 95 : 5.

2. Aqueous dispersion as claimed in claim 1, characterized in that the polymers A and B are emulsion polymers of vinylmonomers.

3. Aqueous dispersion as claimed in claim 2, characterized in that the carboxyl group-containing polymer contains 10 - 70% by weight of units of acrylic and/or methacrylic acid.

4. Aqueous dispersion as claimed in claims 2 and 3, characterized in that the polymers A and B contain units of alkyl esters of acrylic and/or methacrylic acid polymerised therein.

5. Aqueous dispersion as claimed in claims 1 to 4, characterized by a dynamic freezing temperature of the film forming polymer of between 10 and 60°C.

6. Aqueous dispersion as claimed in claims 1 to 5, characterized in that the content of polymers A and B is from 10 - 40% by weight.

7. Use of an aqueous dispersion as claimed in claims 1 to 6 for coating pharmaceutical preparations.

8. Use of an aqueous dispersion as claimed in claims 1 to 6 for producing pharmaceutical capsules.

9. Process for preparing pharmaceuticals coated to render them resistant to gastric juices or to delay their release, by coating with an aqueous pharmaceutical coating dispersion and drying, characterized in that an aqueous dispersion as claimed in claims 1 to 6 is applied.

10. Pharmaceutical preparation containing a coating which contains polymers A and B as claimed in claim 1 in a weight ratio of above 60 : 40 and below 95 : 5.

11. Pharmaceutical preparation as claimed in claim 10, characterized by a coating with a thickness of 10 - 30 µm on granules, particles or crystals.

12. Pharmaceutical preparation as claimed in claim 10, characterized by a coating applied to granules, particles or crystals in an amount of 10 to 20% by weight, based on the weight of the coated pharmaceutical preparations.

13. Pharmaceutical preparation as claimed in claim 10, characterized by a coating applied to tablets, coated tablets or capsules, in an amount of from 3 - 5% by weight, based on the weight of the coated pharmaceutical preparation.

14. Pharmaceutical capsules resistant to gastric juices, consisting of a mixture of polymers A and B as claimed in claim 1 in a weight ratio above 60 : 40 and below 95 : 5.

## Revendications

1. Dispersion aqueuse d'un agent d'enrobage pour médicaments, contenant des particules de latex en dispersion

A) d'un polymère soluble dans l'eau entre pH 5 et 8, contenant des groupes carboxyle, et

B) d'un polymère non-soluble dans l'eau, filmogène, présentant une dilatabilité élevée,

caractérisée en ce que le rapport en poids entre la quantité totale des particules de latex A et B est supérieur à 60 : 40 et inférieur à 95 : 5.

2. Dispersion aqueuse selon la revendication 1, caractérisée en ce que les polymères A et B sont des polymères en émulsion de monomères vinyliques.

3. Dispersion aqueuse selon la revendication 2, caractérisée en ce que le polymère contenant des groupes carboxyle contient de 10 à 70% de motifs acide acrylique et/ou acide méthacrylique (% en poids).

4. Dispersion aqueuse selon les revendications 2 et 3, caractérisée en ce que les polymères A et B contiennent des motifs polmérisés d'esters alkyliques de l'acide acrylique et/ou de l'acide méthacrylique.

5. Dispersion aqueuse selon les revendications 1 à 4, caractérisée par une température dynamique de transition vitreuse du polymère filmogène comprise entre 10 et 60°C.

6. Dispersion aqueuse selon les revendications 1 à 5, caractérisée en ce qu'elle contient de 10 à 40% en poids des polymères A et B.

7. Utilisation d'une dispersion aqueuse selon les revendications 1 à 6 pour l'enrobage de formes médicamenteuses.

8. Utilisation d'une dispersion aqueuse selon les revendications 6 pour la préparation de gélules de médicaments.

9. Procédé pour la préparation de formes médicamenteuses enrobées, gastro-résistantes ou à effet retard, par enrobage à l'aide d'une dispersion aqueuse d'enrobage de médicament et séchage, caractérisé en ce qu'on applique une dispersion aqueuse selon les revendications 1 à 6.

10. Forme médicamenteuse contenant un enrobage, qui contient les polymères A et B selon la revendication 1 selon un rapport pondéral supérieur à 60 : 40 et inférieur à 95 : 5.

11. Forme médicamenteuse selon la revendication 10, caractérisée par un enrobage d'épaisseur 10 à 30 micromètres sur des granules, des particules ou des cristaux.

12. Forme médicamenteuse selon la revendication 10, caractérisée par un enrobage sur des granules, des particules ou des cristaux, avec une application de vernis de 10 à 20% en poids par rapport au poids des formes médicamenteuses enrobées.

13. Forme médicamenteuse selon la revendication 10, caractérisée par un enrobage sur des comprimés, des dragées ou des gélules, avec une application de vernis de 3 à 5% en poids par rapport au poids des formes médicamenteuses enrobées.

14. Gélules de médicaments gastro-résistantes, constituées d'un mélange des polymères A et B selon la revendication 1, selon un rapport pondéral supérieur à 60 : 40 et inférieur à 95 : 5.